# EUROPEAN PATENT SPECIFICATION

(11) **EP 3 540 078 B1**
(45) Date of publication and mention of the grant of the patent: **05.10.2022**
(21) Application number: 18211486.8
(22) Date of filing: 11.12.2018
(51) Int. Cl.: C12Q 1/6886

(54) **METHOD FOR THE EVALUATION OF THE PROGNOSIS OF BREAST CANCER**
VERFAHREN ZUR BEWERTUNG DER PROGNOSE VON BRUSTKREBS
PROCÉDÉ POUR L'ÉVALUATION DU PRONOSTIC DU CANCER DU SEIN

(30) Priority: 11.03.2018 US 201862641385 P
(43) Date of publication of application: 18.09.2019
(73) Proprietor: National Cheng Kung University, Tainan City 701 (TW)
(72) Inventor: Chen, Pai-Sheng, 730 Tainan City (TW); Li, Jie-Ning, 704 Tainan City (TW); Kuo, Yao-Lung, 704 Tainan City (TW)
(74) Representative: Straus, Alexander

(56) References cited:
- US-A1- 2009 181 397
- DONG X Y ET AL: "Implication of snoRNA U50 in human breast cancer", JOURNAL OF GENETICS AND GENOMICS, ELSEVIER LTD, AMSTERDAM, NL, vol. 36, no. 8, 1 August 2009 (2009-08-01) , pages 447-454, XP026469244, ISSN: 1673-8527, DOI: 10.1016/S1673-8527(08)60134-4 [retrieved on 2009-08-01]
- ZURAB SIPRASHVILI ET AL: "The noncoding RNAs SNORD50A and SNORD50B bind K-Ras and are recurrently deleted in human cancer", NATURE GENETICS., vol. 48, no. 1, 1 January 2016 (2016-01-01), pages 53-58, XP055605348, NEW YORK, US ISSN: 1061-4036, DOI: 10.1038/ng.3452 & Zurab Siprashvili ET AL: "The noncoding RNAs SNORD50A and SNORD50B bind K-Ras and are recurrently deleted in human cancer supplementary information", NATURE GENETICS., vol. 48, no. 1, 23 November 2015 (2015-11-23), pages 53-58, XP055605354, NEW YORK, US ISSN: 1061-4036, DOI: 10.1038/ng.3452
- ELENA S. MARTENS-UZUNOVA ET AL: "C/D-box snoRNA-derived RNA production is associated with malignant transformation and metastatic progression in prostate cancer", ONCOTARGET, vol. 6, no. 19, 19 May 2015 (2015-05-19), pages 17430-17444, XP055360293, DOI: 10.18632/oncotarget.4172

### SEQUENCE LISTING

<110> National Cheng Kung University
<120> METHOD AND KIT FOR THE EVALUATION OF THE PROGNOSIS OF BREAST CANCER
<130> NUK-P0011-EPC
<150> US 62/641,385
   <151> 2018-03-11
<160> 3
<170> PatentIn version 3.5
<210> 1
   <211> 75
   <212> RNA
   <213> Homo sapiens
<400> 1
   uaucugugau gaucuuaucc cgaaccugaa cuucuguuga aaaaaaaaaa cuuuuacgga 60
   ucuggcuucu gagau 75
<210> 2
   <211> 31
   <212> DNA
   <213> Homo sapiens
<400> 2
   tatctgtgat gatcttatcc cgaacctgaa c 31
<210> 3
   <211> 22
   <212> DNA
   <213> Homo sapiens
<400> 3
   atctcagaag ccagatccgt aa 22

## Description

The present invention provides The present invention provides a method for the detection of the presence of breast cancer in a subject according to the pre-characterizing clause of claim 1.

Breast cancer is the most common cancer type among women with an increasing incidence rate worldwide and the second leading cause of cancer death. For clinical implications and treatment strategies, immunohistochemistry (IHC) markers, such as ER, PR and HER2, combined with clinicopathological variables, including tumor size, tumor grade and nodal involvement, have been utilized routinely. With the promotion of prevention medicine, breast cancer can be diagnosed in the early stages. However, there is no suitable prognostic marker for the early prediction of breast cancer survival. Furthermore, traditional pathological markers used to classify breast tissues into the TNM stages have limitations for discriminating individual variability. Hence, the need for establishing a molecular marker for early detection is urgent.

Small nucleolar RNAs (snoRNAs) are small noncoding RNAs ranging from 60-300 nucleotides. These snoRNAs are essential for numerous cellular processes and execute their function mainly in the nucleolus. SnoRNAs are classified according to their structures and function into C/D box and H/ACA box types. By binding different associated proteins and enzymes, C/D and H/ACA box snoRNAs become small nucleolar RNA-protein complexes (snoRNPs) and execute site-specific methylation or pseudouridylation of rRNAs guided by snoRNAs. Although they are considered to be housekeeping RNAs, aberrant expression of snoRNAs has been reported recently in human cancers. SNORA42 is elevated in nonsmall cell lung cancer (NSCLC) patients, which is correlated with poor prognosis. SNORD76 expression is lower in grade III/IV patients compared to grade II patients, which is considered as a tumor suppressor in glioblastoma. According to Siprashvili, et. Al, "The noncoding RNAs SNORD50A and SNORD50B bind K-Ras and are recurrently deleted in human cancer "Nature Genetics, volume 48, pages 53-58 (2016), SNORD50 (U50) is a putative tumor suppressor since the deletion of U50 is correlated with poor prognosis in breast cancer patients. Although 20% of breast cancers are heterozygous deletion of theU50 locus in the genome, no homozygous deletion has been observed. U50 snoRNA has a noncanonical function in inactivating KRAS, indicating its tumor-suppressive properties. However, the role and impact of U50 in breast cancer has not been elucidated.

For example, in a first reference (US 2009/181397), the lower U50 expression levels in tumors and the role of U50 as tumor suppressor have been disclosed. However, the first reference does not show nor explicitly teach the expression level of U50 being used as prognostic marker, nor does it disclose or teach the classification of stage breast cancer. In a second reference (Implication of snoRNA U50 in human breast cancer, JOURNAL OF GENETICS AND GENOMICS, ELSEVIER LTD, AMSTERDAM, NL, vol. 36, no. 8, 1, pages 447-454), U50 being a tumor suppressor and having a functional role in breast cancer progression and development are disclosed. However, the second reference does not disclose the expression level of U50 can be used to distinguish patients regarding their survival prognosis and to teach a prognosis of stage 1 breast cancer. In a third reference (The noncoding RNAs SNORD50A and SNORD50B bind K-Ras and are recurrently deleted in human cancer, NATURE GENETICS, vol. 48, no. 1, 1, pages 53-58), SNORD50A being deleted in more than 20% of breast cancer samples, and the deletion of SNORD50A being linked to a worse prognosis in breast cancer, are disclosed. Although disclosing a method to evaluate a breast cancer prognosis, the third reference does not clearly disclose or teach a specific prognosis of stage 1 breast cancer, and a specific staging of breast cancer based on U50 expression levels.

This in mind, the present invention quantified the copy number of snoRNA U50 using absolute quantification real-time PCR in breast cancer patient tissues. To evaluate the potential RNA level of U50 as a prognostic marker, we validated and correlated U50 expression with clinical characteristics, including the stage, TNM stage and survival times.

The present invention therefore provides a method and a kit using snoRNA U50 as a biomarker, which can effectively diagnose the breast cancer.

According to one embodiment, a method for predicting the survival time of a patient suffering from a breast cancer is provided. First, the RNA expression level of snoRNA U50 is measured, in a sample isolated from a subject, After comparing said expression level with a predetermined reference value, a good prognosis is provided when the expression level of the selected snoRNA is higher than the predetermined reference value and a poor prognosis is provided when the expression level of the selected snoRNA is lower than the predetermined reference value, wherein a prognosis of stage 1 breast cancer is given when the RNA expression level of the snoRNA U50 is between 400 copies/ng and 225 copies/ng.

In the following, the invention is further illustrated by way of example, taking reference to the accompanying drawings.
FIG. 1 shows absolute quantification of U50 snoRNA,
FIG. 2 shows the U50 snoRNA copy number in respect to the U50-high group and the U50-low group of the breast cancer patients,
FIG. 3 shows Kaplan-Meier curves for overall survival and relapse-free survival of U50-high and U50-low breast cancer patients,
FIG.4 shows SnoRNA copy numbers of U50 in ER+/- (A), PR+/- (B) and HER2+/- (C) breast cancer patients,
FIG. 5 shows the correlation between U50 snoRNA copy number and pathological stage of breast cancer patients,
FIG. 6 shows the correlation between the U50 snoRNA copy number and clinical T stage (A) and N stage (B) breast cancer patients,
FIG. 7 shows the correlation between the U50 snoRNA copy number and tumor size in breast cancer patients,
FIG. 8 shows Kaplan-Meier curves for the overall survival of patients with tumor sizes >20 mm in U50-high and U50-low breast cancer patients,
FIG. 9 shows relative levels of U50 expression in U50-overexpressing MCF-7 cells, and

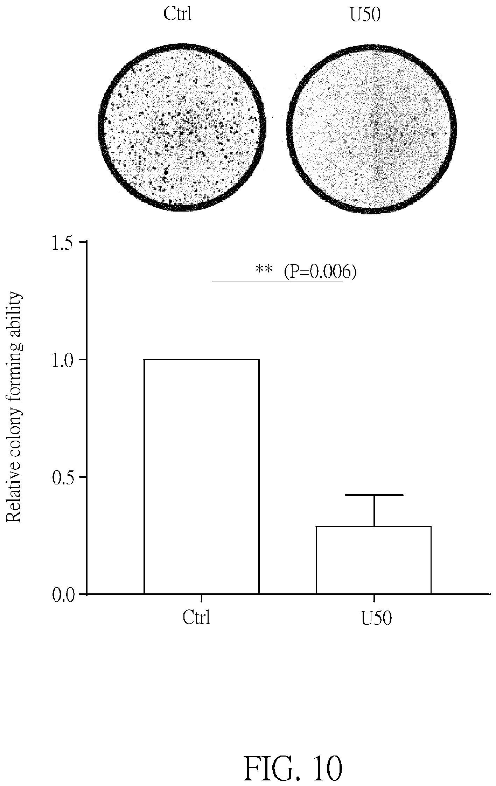

FIG. 10 shows colony-forming ability of MCF-7 cells expressing U50.

To provide a better understanding of the presented invention, preferred embodiments will be made in detail. The preferred embodiments of the present invention are illustrated in the accompanying drawings with numbered elements.

Small nucleolus RNAs (snoRNAs) are small noncoding RNAs that are essential for numerous cellular processes and are generally considered housekeeping genes. In the present invention, the copy numbers of snoRNAU50 (5'- -3', SEQ ID: NO. 1)is quantified in breast cancer patient tissues and found that a higher level of U50 expression is correlated with better overall survival in breast cancer patients, suggesting that U50 is an independent prognostic marker. In addition, the level of U50 was lower in stage 2 and 3 than in stage 1 tumors, whereas no correlation was observed with lymph node metastasis. The present invention has shown the clinical relevance of snoRNA U50 in human breast cancer, which provides important clinical information for further research.

To show the potential of snoRNA U50 as a prognostic marker, the following context will validated and correlated U50 expression with clinical characteristics, including stage, TNM stage and survival times.

### Materials and methods

### Patient specimens

We analyzed 142 samples of breast cancer tissues from individual patients. Specimens were collected from National Cheng Kung university hospital (Tainan, Taiwan) during 2003-2013 from patients 23 to 83 years of age. Frozen breast cancer tissues were used according to approved IRB protocols from NCKU Hospital. Tumor stage was classified according to the American Cancer Society.

### Total RNA extraction

Total RNA was extracted using TRIzol (Invitrogen) according to manufacturer's protocol. Briefly, chloroform was added and homogenized samples were centrifuged at 14000 g for 30 mins. The clear upper aqueous layer was isolated and precipitated with isopropanol at -80°C for 1-2 hours. Samples were centrifuged at 14000 g for 30 mins, and the supernatant was discarded. The resulting pellets were washed with 75% ethanol and preserved in nuclease-free water at -80°C. Total RNA concentrations were determined by spectrophotometer (NanoDrop Lite Microlitre Spectrophotometer).

### Reverse transcription

Reverse transcription was performed using the RevertAid RT Reverse Transcription Kit (Thermo Scientific^{™}) according to the manufacturer's protocol. A reaction containing 200 ng template RNA, 1 µL random hexamer, and 2 µL 10 mM dNTP mix with nuclease-free water adjusted to 12 µL was incubated at 65°C for 5 mins. A mixture of 4 µL 5× reaction buffer, 1 µL RNase inhibitor, and 1 µL RevertAid RT was added, the volume was brought 20 µL to with nuclease-free water, and the reactions were incubated at 25°C for 5 mins, followed by 42°C for 60 mins. Finally, the reactions were terminated at 70°C for 5 mins. cDNAs were preserved at -20°C.

### Absolute real-time PCR

A standard curve was established using linearized U50 overexpressing plasmid. Ten-fold dilutions were used, and the initial concentration was adjusted to 108 copy numbers to achieve the final concentration within 40 cycles. Real-time PCR was performed in 48 well optical plates with four repetitions. Total volume consisted of 2.5 µL Fast SYBR^{™} Green PCR Master Mix, 0.5 µL forward primer, 0.5 µL reverse primer, 0.5 µL nuclease-free water and 1 µL template. The real-time PCR program was carried out according to the manufacturer's protocol, which included 95°C for 20 sec to activate the polymerase, 40 cycles of 95°C for 3 sec and 60°C for 30 sec.

### SnoRNA detection

SnoRNA detection has been described previously (ref). Briefly, the total RNA was extracted and reverse transcription was performed. Samples were further applied to absolute real-time PCR to calculate the copy numbers of snoRNAs. The primers used for detecting U50 have been described previously (ref): forward primer: 5'-TATCTGTGATGATCTTATCCCGAACCTGAAC-3' (SEQ ID: NO. 1) and reverse primer: 5'-ATCTCAGAAGCCAGATCCGTAA-3'(SEQ ID: NO. 2).

### Colony formation

A total of 2000 or 4000 MCF-7/Control and MCF-7/U50 cells were seeded into 6-well plates. After 7-14 days, colonies were formed and fixed with 3.7% paraformaldehyde at room temperature for 30 mins. The fixed colonies were stained by 0.05% Coomassie blue for 15 mins at room temperature. The colonies in every well were counted using ImageJ software.

### Plasmids and transfection

Cells were seeded a ∼ ~80% confluency. Expression plasmids for snoRNA U50 (pSIREN-RetroQ-U50) were a kind gift from Dr. Dong. The plasmids were transfected into MCF-7 cells using the HyFectTM DNA transfection reagent according to the manufacturer's protocol (Leadgene Biomedical, Taiwan). Briefly, 6 µg of plasmid was mixed with the cells and incubated for 25 mins at room temperature. The cells were then added to dishes with fresh medium and incubated for 48 hours.

### Statistical analysis

We used a log-rank test in Kaplan-Meier curves for overall and relapse-free survival. A t-test was used to compare U50 expression with ER, PR, HER2 and the tumor size. For multigroup analysis, one-way ANOVA with Tukey's multiple comparisons test was used.

### Result

### Absolute quantitative real-time PCR for snoRNA U50 in breast cancer patient tissues.

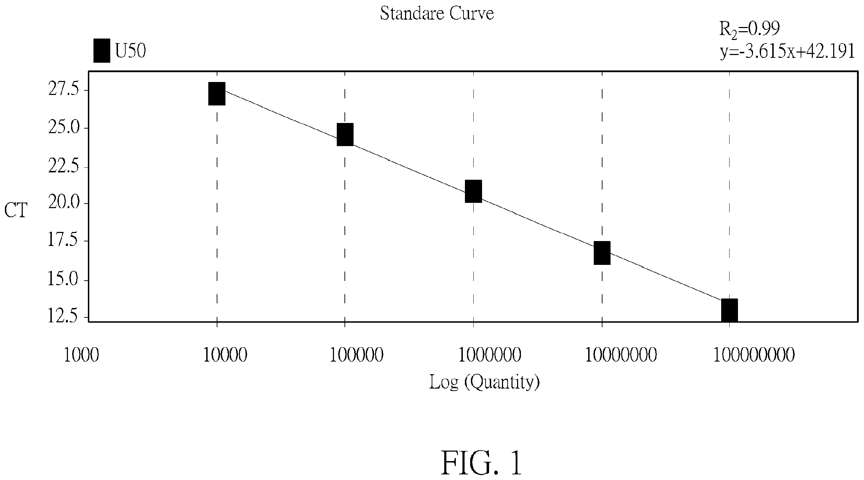

We used absolute quantitative real-time PCR to quantify U50 snoRNA copies numbers. A ten-fold serial dilution of linearized plasmid template was performed, and the initial concentration was adjusted so all the dilution points could be detected within 40 C_{T} cycles. The result is shown in FIG. 1, wherein the standard curve was confirmed from 10⁴ to 10⁸ U50 copy numbers. The R²value was 0.99 for U50.

### Validation of absolute U50 expression as a prognostic marker in breast cancer patients

We analyzed 142 samples of breast cancer tissues from individual patients. The clinical and pathological characteristics of patients are listed in Table 1.

| **Table 1. Baseline characteristics in the study.** | |
|---|---|
| Characteristics | No. of patients (%) |
| Median age (range, years) | 54 (23 - 84) |
| Age 50 | 62 (43.7%) |
| Age 50 | 80 (56.3%) |
| Pathologic characteristic | |
| Invasive ductal carcinoma | 130 (91.5) |
| Invasive lobular carcinoma | 2 (1.43) |
| Others | 10 (7.14) |

| | |
|---|---|
| _{†}Others: Mucinous carcinoma, papillary carcinoma and invasive carcinoma | |

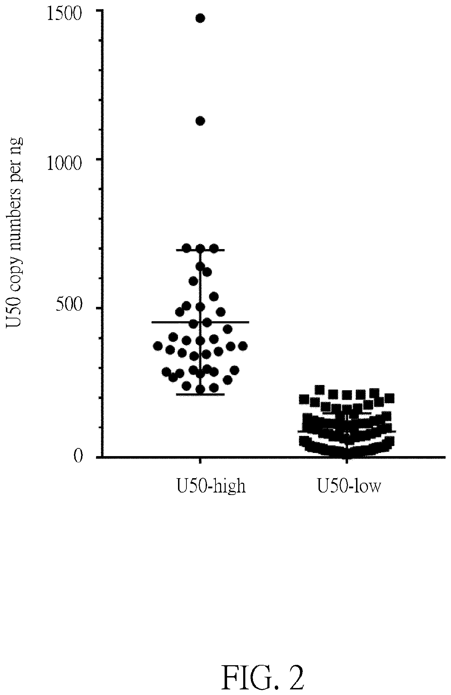

First, we used the Cutoff Finder (http://molpath.charite.de/cutoff/), which determines of cutoff point of molecular markers to determine the optimal cutoff point. We divided breast cancer patients into two groups using cutoff point: U50-high and U50-low by using RNA expression level of 227.5 copies/ng as a cutoff number in the present embodiment. Next, we investigated the correlation between U50 snoRNA copy numbers and breast cancer patient survival and found that higher expression of U50 was statistically and significantly correlated with better overall survival in breast cancer patients (P=0.001, HR=0.17; FIG. 2A). Longer relapse-free survival was also observed in breast cancer patients with elevated U50 expression (P=0.002, HR=0.2; FIG. 2B). In another embodiment, the cutoff number between 340 copies/ng and 101.88 copies/ng can also give significant relationship (P<0.05) between the RNA expression level and the prognosis of breast cancer. These data indicated that U50 copy number (RNA expression level) is significantly predictive of breast cancer patient survival. The cutoff number can uses as a reference value to determine the prognosis of the breast cancer: when the RNA expression level of snoRNA U50 of a patient is higher than the predetermined reference value, a good prognosis will be given; when the RNA expression level of the snoRNA U50 is lower than the predetermined reference value, a poor prognosis will be given. It is understood that the cutoff value (the predetermined reference value), for example, can be adjusted according to other clinical results depending on different countries or races. The cutoff number interval 350 copies/ng~100 copies/ng only shows one preferred embodiment and should not limit the claim scope.

### Associations of U50 expression with ER, PR, and HER2

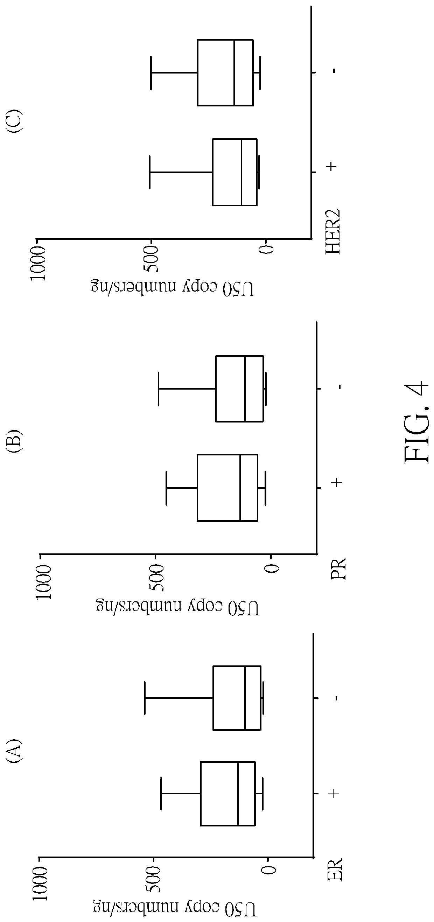

Since ER, PR and HER2 expression demonstrate distinct morphologies and clinical implications in breast cancer patients, we analyzed the correlation between U50 expression and breast cancer subtypes. In ER/PR/HER2-positive and ER/PR/HER2-negative breast cancer patients, U50 expression was not significantly different (P=0.42, 0.21 and 0.85 in ER, PR and HER2 groups, respectively; FIG. 4), suggesting that U50 copy number is independent of molecular subtype. The results of the detailed analyses including the numbers of each group, mean ± S.E. and range are shown in Table 2.

**Table 2. Expression of ER, PR and HER2 and snoRNA copy numbers of U50**

| Receptor | Numbers | Mean ± S.E. | Range | *P* value |
|---|---|---|---|---|
| **Estrogen receptor (ER)** | 0.42 | | | |
| Positive | 95 | 209.5 ± 23.79 | 162.2 - 256.7 | |
| Negative | 34 | 173.5 ± 32.45 | 107.4 - 239.5 | |
| **Progesterone** | 0.21 | | | |
| **receptor (PR)** | | | | |
| Positive | 69 | 222.8 ± 30.34 | 162.2 - 283.3 | |
| Negative | 60 | 173.7 ± 22.98 | 127.7 - 219.7 | |
| **Human** | | | | |
| **Epidermal** | | | | |
| **Growth** | 0.85 | | | |
| **Factor Receptor 2 (HER2)** | | | | |
| Positive | 24 | 201.6 ± 62.88 | 71.56 - 331.7 | |
| Negative | 91 | 211.5 ± 21.65 | 168.5 - 254.5 | |

| | | | | |
|---|---|---|---|---|
| S.E.: Standard error of the mean. #Calculated using the unpaired t test. | | | | |

### Associations between the pathological characteristic markers of breast cancer patients and U50 expression

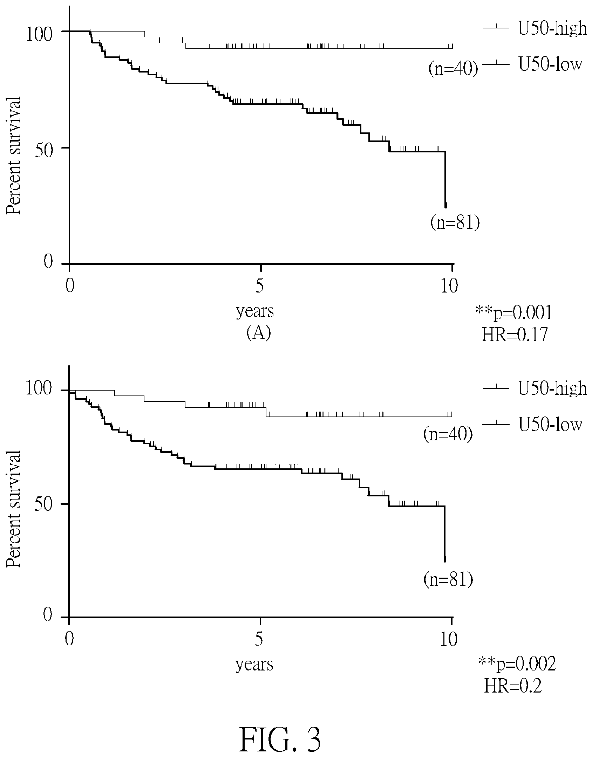

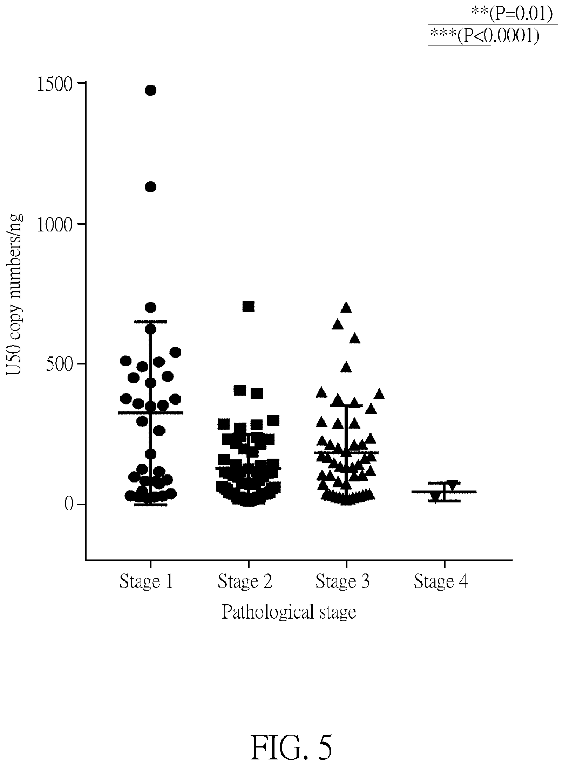

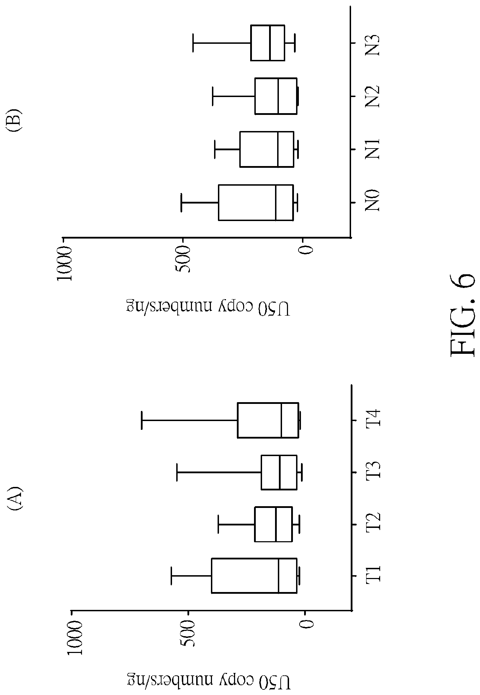

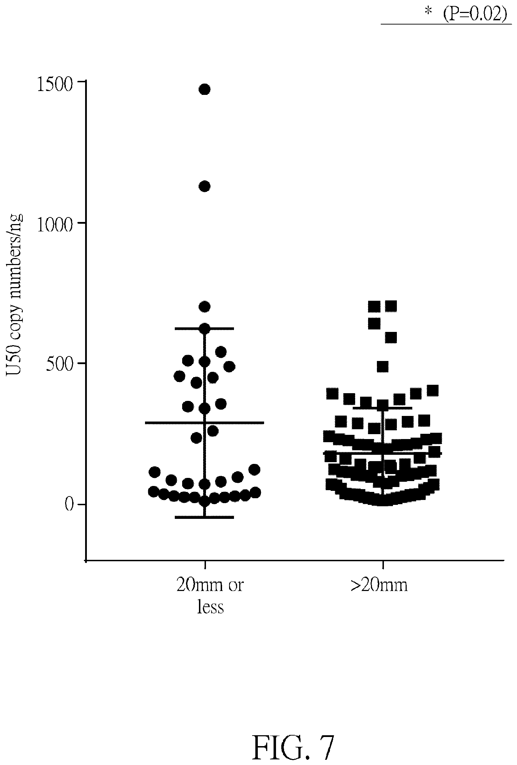

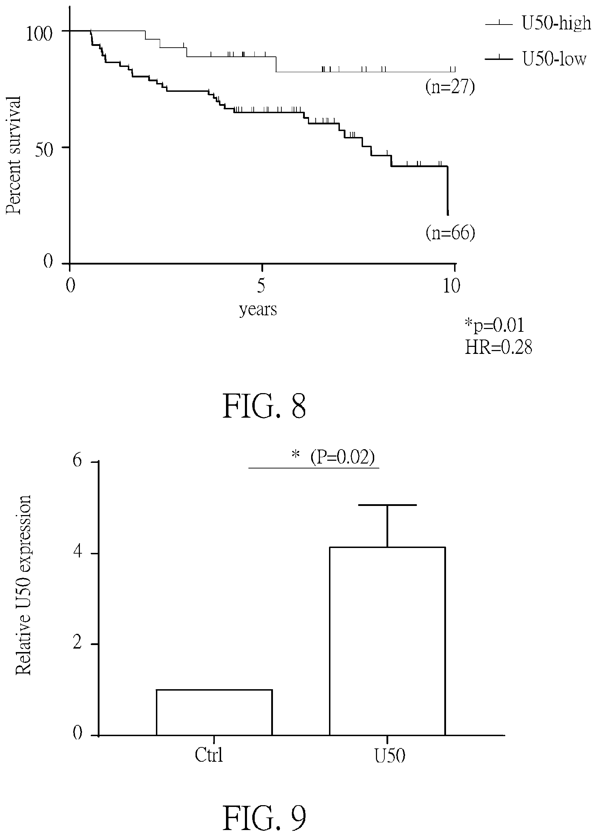

Since U50 can be used to predict breast cancer patient survival (FIG. 3) considered as a prognostic marker, we next investigated the relationship between U50 expression and pathological parameters. In breast cancer patient tissues, U50 copy numbers were dramatically lower in stage 2 and 3 cancers than in stage 1 (P<0.0001 and *P*=0.01, respectively), indicating that U50 copy numbers are reduced in late stage breast cancers (FIG. 5 and Table 3). U50 expression showed no difference in the clinical T and N categories (P=0.136 and 0.366, respectively; FIG. 6 and Table 4). However, tumors ≦ 20 mm in diameter had higher U50 expression than tumors > 20 mm (P=0.02; FIG. 7 and Table 5). Expression of U50 can predict the overall survival in breast cancer patients with tumors > 20 mm (FIG.8). These results indicated that high U50 expression was observed in the early stages of breast cancer, and during breast tumor progression, the U50 copy number is downregulated resulting in a decline in tumor-suppression activity and contributing to tumorigenesis.

**Table 3. Correlation between snoRNA copy numbers of U50 and pathological stage of breast cancer patients**

| Stage | I | II | III | IV |
|---|---|---|---|---|
| Numbers(%) | 33 (22.9) | 59 (40.9) | 50 (34.7) | 2 (1.38) |
| Mean ± S.E. | 323.6 ± 56.81 | 124.8 ± 15.97 | 181.7 ± 23.74 | 42.18 ± 22.21 |
| Minimum | 21.13 | 9.74 | 14.15 | 19.97 |
| 25% percentile | 74.87 | 37.41 | 35.16 | 19.97 |
| Median | 293.1 | 96.87 | 136.4 | 42.18 |
| 75% percentile | 470.2 | 184.5 | 246.8 | 64.38 |
| Maximum | 1475 | 701.7 | *700.5* | 64.38 |
| Std. Error of Mean | 56.81 | 15.97 | 23.74 | 22.21 |
| *P* value | 0.0001 | | | |

| | | | | |
|---|---|---|---|---|
| S.E.: Standard error of the mean. * Stage according to the recommendations of the American Cancer Society | | | | |

**Table 4. Correlation between snoRNA copy numbers of U50 and T/N category of breast cancer patients**

| T category | Numbers | Mean ± S.E. | *P* value |
|---|---|---|---|
| **T1 (%)** | 45(32.1) | 252.2 ± 45.26 | 0.136 |
| **T2 (%)** | 73 (52.1) | 158.6 ± 16.01 | |
| **T3 (%)** | 15 (10.8) | 165.8 ± 46.22 | |
| **T4 (%)** | 7 (5) | 206.1 ±90.59 | |

| N category | Numbers | Mean ± S.E. | |
|---|---|---|---|
| **NO (%)** | 61 (43) | 224.9 ± 34.41 | 0.366 |
| **N1 (%)** | 32 (22.5) | 170.2 ± 31.01 | |
| **N2 (%)** | 27(19) | 142.8 ± 28.23 | |
| **N3 (%)** | 22 (15.5) | 185.9 ± 33.36 | |

| | | | |
|---|---|---|---|
| S.E.: Standard error of the mean. ∗Stage according to the recommendations of the American Cancer Society _{#}Use One-way ANOVA, Tukey's multiple comparisons test | | | |

**Table 5. Correlation between snoRNA copy numbers of U50 and tumor size of breast cancer patients**

| Tumor size | Numbers | Mean ± S.E. | *P* value |
|---|---|---|---|
| 20mm | 34 (30.6) | 287.9 ± 57.43 | 0.02 |
| 20mm | 77 (69.4) | 179.5 ± 18.36 | |

| | | | |
|---|---|---|---|
| S.E.: Standard error of the mean. _{#}Use unpaired t test | | | |

### The function of U50 in human breast cancer cells

We have shown that U50 is a prognostic marker for early breast cancer detection. Since U50 expression is decreased in patients with tumors > 20 mm (FIG. 6), we asked whether U50 is negative regulator of tumorigenesis. We used MCF-7 as a cell model and established stable U50-overexpressing cells. In U50-overexpressing MCF-7 cells, U50 expression was confirmed to be four-fold higher than the control cells (FIG. 8). Next, we performed colony formation assays to evaluate the effect of U50 on tumorigenesis in MCF-7 cells. Decreased colony forming ability in U50-overexpressing MCF-7 cells was observed, suggesting that U50 functions as a tumor suppressor and inhibits tumorigenesis in breast cancer cells (FIG. 9). The *in vitro* results showed that U50 is a negative regulator of tumorigenesis, which is consistent with the clinical patient results showing that U50 copy number is higher in early stages of breast cancer and in tumors smaller than 20 mm.

Since there is no study indicating the relationship between the snoRNA U50 and the breast cancer in an RNA level, the present invention has identified the prognostic value and molecular function of RNA expression of snoRNA U50 in breast cancer. Accordingly, the present invention provides a method for detection of the presence of or the risk of breast cancer in a subject. First, the RNA expression level of snoRNA U50 is detected, in a sample isolated from a subject. In one embodiment, the subject refers to patient or person suspected to have breast cancer and can be male or female. Sample is prepared from frozen breast tissue. After carrying out a total RNA extraction process, the expression level of snoRNA U50 is detected by calculating the number of snoRNA U50 a quantities RT-PCR. In one embodiment, the primer in RT-PCR is shown in SEQ ID: NO. 2 and SEQ ID: NO. 3. The result is compared to a normal control, wherein a decreased expression level of the snoRNA with reference to a normal control indicates the presence of or the risk of breast cancer in the patient from whom the sample was isolated. In one embodiment, the normal control value is between 350 copies/ng and 100 copies/ng. In another embodiment, the expression level of snoRNA U50 between 400 copies/ng and copies/225 ng (preferably between 383.11 copies/ng and 269.49 copies/ng) indicates the stage 1 of breast cancer, the expression level of snoRNA U50 between 225 copies/ng and 100 copies/ng (preferably between 205.44 copies/ng and 157.96 copies/ng) indicates the stage 2 or stage 3 of breast cancer, the expression level of snoRNA U50 less than 100 copies/ng (preferably between 64.39 copies/ng and 19.97 copies/ng) indicates the stage 4 of breast cancer.

The present invention further provides a method for predicting the survival time of a patient suffering from a breast cancer. First, measuring the expression level of snoRNA U50, in a sample isolated from a subject. Next, said expression level with a predetermined reference value is measured. According to the result, a good prognosis is provided when the expression level of the selected snoRNA is higher than the predetermined reference value and a poor prognosis when the expression level of the selected snoRNA is lower than the predetermined reference value.

The present invention also provides a kit for the detection of the presence of or the risk of breast cancer in a subject. The kit contains components required for detecting the RNA expression of snoRNA U50 in a sample, such as Northern blot analysis, RT-PCR, microarray analysis or sequencing analysis from an object. In one embodiment, the kit contains components for performing quantitative RT-PCR, including: nucleotides: a reverse transcriptase, a polymerase, a forward primer (SEQ ID: NO. further include assay containers (tubes), buffers, or enzymes necessary for carrying out the detection assay. In one embodiment, the kit can shows if the RNA expression level U50 of a subject is higher than a predetermined value, for example, by showing a bar, a signal or other readable forms to give an evaluation of prognosis of breast cancer.

In summary, U50 expression can be used to differentiate breast cancer patient survival. Higher expression of U50 is correlated with better overall survival and relapse-free survival, which is a prognostic marker in breast cancer. We also revealed that U50 is an independent molecular marker for discriminating early stage breast cancer. Along with tumor progression, we demonstrated the tumor suppression activity of U50 in inhibiting tumorigenesis, which is consistent with the clinical patient data. In this study, we established a new molecular marker, U50, for breast cancer early detection and prognosis prediction.

## Claims

1. A method for evaluating the prognosis of a patient suffering from breast cancer, **characterized by** comprising the steps:
measuring an RNA expression level of snoRNA U50 **characterized by** the nucleotide sequences identified herein as SEQ ID No. 1, in a sample isolated from the patient;
comparing said RNA expression level with a predetermined reference value; and
providing a good prognosis when the RNA expression level of the snoRNA U50 is higher than the predetermined reference value and a poor prognosis when the RNA expression level of the snoRNA U50 is lower than the predetermined reference value, wherein a prognosis of stage 1 breast cancer is given when the RNA expression level of the snoRNA U50 is between 400 copies/ng and 225 copies/ng.

2. The method according to claim 1, **characterized in that** detection is carried out by amplifying the gene using quantitative real-time PCR forward primer used in quantitative real-time PCR is shown in SEQ ID: NO.2 and reverse primer is shown in SEQ ID: NO.3.

3. The method according to claim 1, **characterized in that** the predetermined reference value is between 350 copies/ng and 100 copies/ng.

4. The method according to claim 1, **characterized in that** a prognosis of stage 2 or stage 3 breast cancer is given when the RNA expression level of the snoRNA U50 is between 225 copies/ng and 100 copies/ng.

5. The method according to claim 1, **characterized in that** a prognosis of stage 4 breast cancer is given when the RNA expression level of the snoRNA U50 is less than 100 copies/ng.

## Patentansprüche

1. Verfahren zur Bewertung der Prognose eines an Brustkrebs erkrankten Patienten, **dadurch gekennzeichnet, dass** es die Schritte umfasst:
Messen eines RNA-Expressionslevels von snoRNAU50, das durch die hier als SEQ ID Nr. 1 identifizierten Nukleotidsequenzen charakterisiert ist, in einer von dem Patienten isolierten Probe;
Vergleichen des RNA-Expressionslevels mit einem bestimmten Referenzwert; und
Bereitstellen einer guten Prognose, wenn das RNA-Expressionslevel der snoRNA U50 höher ist als der bestimmte Referenzwert, und einer schlechten Prognose, wenn das RNA-Expressionslevel von snoRNA U50 niedriger ist als der bestimmte Referenzwert, wobei eine Prognose für Brustkrebs im Stadium 1 gegeben ist, wenn das RNA-Expressionslevel von snoRNA U50 zwischen 400 Kopien/ng und 225 Kopien/ng liegt.

2. Verfahren nach Anspruch 1, **dadurch gekennzeichnet, dass** die Erfassung durch Amplifikation des Gens unter Verwendung des quantitativen *realtime*-PCR-Vorwärtsprimers, der in SEQ ID: NO.2 gezeigt ist, und des Rückwärtsprimers, der in SEQ ID: NO.3 gezeigt ist, durchgeführt wird.

3. Verfahren nach Anspruch 1, **dadurch gekennzeichnet, dass** der bestimmte Referenzwert zwischen 350 Kopien/ng und 100 Kopien/ng liegt.

4. Verfahren nach Anspruch 1, **dadurch gekennzeichnet, dass** eine Prognose für Brustkrebs im Stadium 2 oder 3 gegeben ist, wenn das RNA-Expressionslevel von snoRNA U50 zwischen 225 Kopien/ng und 100 Kopien/ng liegt.

5. Verfahren nach Anspruch 1, **dadurch gekennzeichnet, dass** eine Prognose für Brustkrebs im Stadium 4 gegeben ist, wenn das RNA-Expressionslevel von snoRNA U50 weniger als 100 Kopien/ng beträgt.

## Revendications

1. Procédé pour évaluer le pronostic d'un patient souffrant d'un cancer du sein, **caractérisé en ce qu'**il comprend les étapes consistant à:
mesurer un niveau d'expression d'RNA du snoRNA U50 **caractérisé par** les séquences nucléotidiques identifiées ici comme SEQ ID No. 1, dans un échantillon isolé du patient;
comparer ledit niveau d'expression d'RNA avec une valeur de référence prédéterminée; et
fournir un bon pronostic lorsque le niveau d'expression de l'RNA du snoRNA U50 est supérieur à la valeur de référence prédéterminée et un mauvais pronostic lorsque le niveau d'expression de l'RNA du snoRNA U50 est inférieur à la valeur de référence prédéterminée, dans lequel un pronostic de cancer du sein de stade 1 est donné lorsque le niveau d'expression de l'RNA du snoRNA U50 est compris entre 400 copies/ng et 225 copies/ng.

2. Procédé selon la revendication 1, **caractérisé en ce que** la détection est effectuée en amplifiant le gène en utilisant une PCR quantitative en temps réel; l'amorce directe utilisée dans la PCR quantitative en temps réel est présentée dans la SEQ ID: NO.2 et l'amorce inverse est présentée dans la SEQ ID: NO.3.

3. Procédé selon la revendication 1, **caractérisé en ce que** la valeur de référence prédéterminée est comprise entre 350 copies/ng et 100 copies/ng.

4. Procédé selon la revendication 1, **caractérisé en ce qu'**un pronostic de cancer du sein de stade 2 ou de stade 3 est donné lorsque le niveau d'expression de l'RNA du snoRNA U50 est compris entre 225 copies/ng et 100 copies/ng.

5. Procédé selon la revendication 1, **caractérisé en ce qu'**un pronostic de cancer du sein de stade 4 est donné lorsque le niveau d'expression de l'RNA du snoRNA U50 est inférieur à 100 copies/ng.
